(19) Europäisches Patentamt
European Patent Office
Office européen des brevets

(11) Veröffentlichungsnummer: **0 207 350**
**B1**

(12) # EUROPÄISCHE PATENTSCHRIFT

(45) Veröffentlichungstag der Patentschrift: **18.01.89**

(51) Int. Cl.⁴: **C 07 C 148/00**, C 07 C 149/40, C 07 C 149/30

(21) Anmeldenummer: **86108155.2**

(22) Anmeldetag: **14.06.86**

(54) **Verfahren zur Herstellung von Diarylsulfiden.**

(30) Priorität: **05.07.85 DE 3524095**

(43) Veröffentlichungstag der Anmeldung:
**07.01.87 Patentblatt 87/2**

(45) Bekanntmachung des Hinweises auf die Patenterteilung:
**18.01.89 Patentblatt 89/3**

(84) Benannte Vertragsstaaten:
**CH DE FR GB LI**

(56) Entgegenhaltungen:
**EP-A- 0 063 327**
**DE-A- 3 309 142**
**GB-A- 2 119 364**

**HOUBEN-WEYL "Methoden der Organischen Chemie", 4. Auflage, Band IX, 1955, GEORG THIEME VERLAG, Stuttgart**

(73) Patentinhaber: **CASSELLA Aktiengesellschaft, Hanauer Landstrasse 526, D-6000 Frankfurt am Main 61 (DE)**

(72) Erfinder: **Bauer, Wolfgang, Dr., Masurenstrasse 6, D-6457 Maintal 3 (DE)**
Erfinder: **Langer, Manfred, Dr., Birsteiner Strasse 47, D-6000 Frankfurt am Main 61 (DE)**
Erfinder: **Sperling, Winfried, Im Feldchen 12, D-6369 Nidderau 2 (DE)**

(74) Vertreter: **Urbach, Hans-Georg, Dr. et al, Hanauer Landstrasse 526, D-6000 Frankfurt am Main 61 (DE)**

**EP 0 207 350 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Diarylsulfiden durch Umsetzung eines aromatischen Diazoniumsalzes mit einem Arenthiol im alkalischen Medium in Anwesenheit eines Katalysators.

Bei einem der gebräuchlichsten Verfahren zur Herstellung von Diarylsulfiden wird die Lösung eines aromatischen Diazoniumsalzes mit einem Arenthiol in alkalischer Lösung umgesetzt (vgl. Houben-Weyl «Methoden der Organischen Chemie», G. Thieme Verlag, Stuttgart, 4. Auflage, (1955), Band IX, 116-117). Die Reaktion nimmt dabei folgenden Verlauf:

$$1.\quad \underset{(I)}{Ar^1N_2Cl} + \underset{(II)}{Ar^2SNa} \longrightarrow \underset{(III)}{Ar^1N_2SAr^2} + NaCl$$

$$2.\quad \underset{(III)}{Ar^1N_2SAr^2} \longrightarrow \underset{(IV)}{Ar^1SAr^2} + N_2$$

In der ersten Stufe werden dabei zunächst die Diaryldiazosulfide III gebildet, die manchmal selbst in wässriger Lösung explosionsartig zerfallen können.

Zur Vermeidung des Sicherheitsrisikos, das durch die mögliche explosionsartige Zersetzung der Diaryldiazosulfide III gegeben ist, sind zwei verschiedene Durchführungsarten der Reaktion bekannt (vgl. Houben-Weyl loc. cit.).

Bei der ersten Methode zersetzt man das Diaryldiazosulfid bereits kurz nach seiner Bildung, indem man die Diazoniumsalzlösung langsam und unter kräftigem Rühren in die über 70°C erwärmte alkalische Lösung des Thiols einfliessen lässt.

Der Nachteil dieser Methode liegt in der bei erhöhter Temperatur eintretenden Zersetzungsreaktion der Arendiazoniumsalze zu Phenolen, wodurch ein merklicher Rückgang in der Ausbeute an Diarylsulfiden eintritt. Ferner resultiert eine hohe Umweltbelastung bzw. eine Belastung der Fabrikationsabwässer durch phenolische Verbindungen, die durch kostenintensive Reinigungsmethoden entfernt werden müssen.

Nach der zweiten Methode führt man die Reaktion bei tieferer Temperatur und in Gegenwart von Kupferpulver durch, das die Zersetzung des Diaryldiazosulfids der Formel III katalysiert. Bei dieser Methode wird zwar die konkurrierende Phenol-Bildung zurückgedrängt oder unterdrückt, es treten jedoch Probleme durch den Umgang mit Kupfer und eine Abwasserbelastung durch Kupfersalze auf.

Der vorliegenden Erfindung lag die Aufgabe zugrunde, ein Verfahren zur Herstelung von Diarylsulfiden anzugeben, das die Nachteile der bisherigen Verfahren vermeidet, insbesondere bessere Ausbeuten erzielt und keine ökologischen und sicherheitstechnischen Probleme aufweist.

Die Lösung dieser Aufgabe gelingt überraschenderweise dadurch, dass man bei der Umsetzung eines aromatischen Diazoniumsalzes mit einem Arenthiol im alkalischen Medium eine nichtmetallische Festsubstanz mit einer spezifischen Oberfläche von gleich oder grösser als 0,02 $m^2/cm^3$, vorzugsweise von gleich oder grösser als 0,06 $m^2/cm^3$, als Katalysator einsetzt.

Solche als Katalysatoren bei dem erfindungsgemässen Verfahren geeignete, nichtmetallische Festsubstanzen mit einer spezifischen Oberfläche von gleich oder grösser als 0,02 $m^2/cm^3$, vorzugsweise von gleich oder grösser als 0,06 $m^2/cm^3$, sind z.B. feinteilige nichtmetallische Feststoffe mit Teilchendurchmessern von gleich oder kleiner als 0,3 mm, vorzugsweise von gleich oder kleiner als 0,1 mm. Geeignet sind z.B. alle wasserunlöslichen synthetischen oder natürlichen Stoffe, die als Adsorptionsmittel bei technischen Adsorptionsverfahren oder bei der Adsorptionschromatographie eingesetzt werden, so z.B.: Aktivkohlen, Tier- und Knochenkohlen, Silikate und Aluminiumsilikate, wie z.B. Siliciumdioxid (Kieselgel), Kieselgur, Bleicherden, Bentonite, Montmorillonit, Aluminiumoxide, Bauxite, Talk, Glaspulver, Molekularsiebe. Ferner sind z.B. geeignet in Wasser unlösliche Oxide und Salze, z.B. Carbonate, Silikate oder Sulfate von Erdalkalimetallen, Zink und dgl, wie z.B. Magnesiumoxid, Magnesiumcarbonat, Magnesiumsilicat, Zinkcarbonat, Calciumcarbonat, Bariumsulfat etc. Derartige, normalerweise als Adsorptionsmittel benutzte Stoffe besitzen zum Teil hohe spezifische Oberflächen von normalerweise 100 $m^2/cm^3$ und mehr. Sie können bei Vorhandensein einer hohen inneren Oberfläche als Katalysator bei dem erfindungsgemässen Verfahren nicht nur in feinteiliger Form, sondern auch in gekörnter Form eingesetzt werden. Dies ist z.B. bei Aktivkohle möglich, die als Pulverkohle und als sogenannte Kornkohle und Formkohle mit Korngrössen von z.B. bis 4 mm im Handel ist.

Bei dem erfindungsgemässen Verfahren können auch feinteilige Stoffe als Katalysatoren verwendet werden, die z.B. normalerweise als Filterhilfsmittel benutzt werden. Typische Perlit-Filterhilfsmittel besitzen z.B. spezifische Oberflächen von ca. 0,35 $m^2/cm^3$. Geeignete Filterhilfsmittel sind unter verschiedenen Bezeichnungen, wie z.B. ®Celite und ®Corlite im Handel.

Als Katalysator bei dem erfindungsgemässen Verfahren können aber auch noch andere Feststoffe, wie z.B. synthetische oder natürliche Ionenaustauscher, insbesondere Kationenaustauscher, verwendet werden.

Die als Katalysator eingesetzten nichtmetallischen Feststoffe sollen unter den Reaktionsbedingungen unlöslich bzw. inert sein.

Die bei dem erfindungsgemässen Verfahren als ein Ausgangsprodukt benötigten aromatischen Diazoniumsalze werden in an sich bekannter Weise im mineralsauren Medium durch Diazotierung eines aromatischen Amins der Formel V

$$Ar^1NH_2 \qquad (V)$$

hergestellt. Der Arylrest $Ar^1$ stellt dabei einen gegebenenfalls ein- oder mehrfach substituierten Arylrest, insbesondere einen gegebenenfalls ein- oder mehrfach substituierten Phenylrest dar. Der Phenylrest kann z.B. ein oder mehrfach durch Alkyl, insbesondere mit 1 bis 4 C-Atomen, Alkoxy, insbesondere mit 1 bis 4 C-Atomen, Halogen, insbesondere Chlor, Brom oder Fluor, Carboxyl, Alkoxycarbonyl, insbesondere mit 1 bis 4 C-Atomen im Alkoxyrest,

und/oder Nitro substituiert sein. Bei einer Mehrfachsubstitution ist dabei in der Regel jedoch nur eine Nitrogruppe vorhanden.

Geeignete Amine der Fomel V sind beispielsweise: Anilin, 2-, 3- oder 4-Methylanilin, 2-, 3- oder 4-Ethylanilin, 2-, 3- oder 4-Isopropylanilin, 2-, 3- oder 4-tert. Butylanilin, 2-, 3- oder 4-Methoxyanilin, 2-, 3- oder 4-Propoxyanilin, 2-, 3- oder 4-Isobutoxyanilin, 2-, 3- oder 4-Chlor-, Brom- oder Fluoranilin, 2,3-, 2,4-, 2,5- oder 2,6-Dichloranilin, 2-Chlor-4-brom-anilin, 2-, 3- oder 4-Aminobenzoesäure, die Methyl-, Ethyl-, Isopropyl- oder Butylester der 2-, 3- oder 4-Aminobenzoesäure, 4-, 5- oder 6-Chlor-2-aminobenzoesäure, 3-, 4-, 5- oder 6-Fluor-2-aminobezoesäure, 2-Amino-4,5-dimethoxy- oder -diethoxy-benzoesäure.

Die Lösung des auf bekannte Weise hergestellten aromatischen Diazoniumsalzes wird in der Regel sofort weiterverarbeitet und in einem alkalischen Medium mit dem Arenthiol in Anwesenheit einer nichtmetallischen Festsubstanz mit einer spezifischen Oberfläche von gleich oder grösser als 0,02 $m^2/cm^3$ zur Umsetzung gebracht.

Normalerweise werden dabei Arenthiol und Katalysator in einem alkalischen Medium vorgelegt und die Lösung des Arendiazoniumsalzes langsam zudosiert.

Die Reaktion wird in einem wässrigen Medium durchgeführt. Als Reaktionsmedium dient üblicherweise Wasser; es können zusätzlich zu dem Wasser jedoch auch mit Wasser mischbare Lösungsmittel, beispielsweise ein- oder mehrwertige Alkohole, insbesondere solche mit 1 bis 4 C-Atomen, wie z.B. Methanol, Isopropanol, Butanol, Isobutanol oder mit Wasser schwer mischbare Lösungsmittel, wie z.B. Methylenchlorid, Toluol oder Chlorbenzol, zugegen sein. Als Arenthiol werden Verbindungen der allgemeinen Formel VI

$$Ar^2SH \qquad (VI)$$

eingesetzt, wobei der Arylrest $Ar^2$ einen gegebenenfalls ein- oder mehrfach substituierten Arylrest, insbesondere einen gegebenenfalls ein- oder mehrfach substituierten Phenylrest darstellt. Der Phenylrest kann z.B. ein- oder mehrfach durch Alkyl, insbesondere mit 1 bis 4 C-Atomen, Alkoxy, insbesondere mit 1 bis 4 C-Atomen, durch Halogen, insbesondere Chlor, Brom, Fluor, und/oder Nitro substituiert sein. Bei einer Mehrfachsubstitution ist dabei in der Regel jedoch nur eine Nitrogruppe vorhanden.

Die Reste $Ar^1$ und $Ar^2$ können auch gleich sein.

Geeignete Arenthiole der Formel VI sind z.B. Benzolthiol, 2-, 3- oder 4-Toluolthiol, 2-, 3- oder 4-Ethylbenzolthiol, 2-, 3- oder 4-Isopropylbenzolthiol, 2-, 3- oder 4-Chlorbenzolthiol, 2-, 3- oder 4-Brombenzolthiol, 2-, 3- oder 4-Fluorbenzolthiol, 2-, 3- oder 4-Nitrobenzolthiol, 2-, 3- oder 4-Methoxybenzolthiol, 2-, 3- oder 4-Propoxybenzolthiol, 2,3-, 2,4- 2,5-Dichlorbenzolthiol, 4-Chlor-2,5-dimethyl- oder -diethyl-benzolthiol.

Das Arenthiol der Formel VI wird im alkalischen Medium in das entsprechende Alkalisalz überführt. Zur Einstelllung der alkalischen Reaktion wird dem wässrigen Medium ein geeignetes Alkali, insbesondere ein Alkalihydroxid, zugesetzt. Als Alkalihydroxide kommen z.B. Lithium-, Natrium- und/oder Kaliumhydroxid in Betrracht, von denen das Natriumhydroxid aus Kostengründen bevorzugt wird.

Dem wässrigen Reaktionsmedium wird soviel Alkali zugesetzt, dass die gesamte Reaktion im alkalischen pH-Bereich, insbesondere in einem pH-Bereich über 8, vorzugsweise im pH-Bereich von 9 bis 14, durchgeführt werden kann.

Die Umsetzung wird normalerweise in einem Temperaturbereich von -10°C bis 50°C, vorzugsweise im Temperaturbereich von 0 bis 20°C, und ganz besonders bevorzugt im Temperaturbereich von 2 bis 10°C, durchgeführt.

In der Regel wird bei der Umsetzung zwischen dem Arenthiol der Formel VI und dem aromatischen Diazoniumsalz ein Molverhältnis von 1 : (1 bis 1,05) eingehalten. Normalerweise werden pro Mol des aromatischen Diazoniumsalzes 5 bis 60 g des erfindungsgemäss verwendeten Katalysators eingesetzt. Höhere Katalysatormengen bringen üblicherweise keine Verbesserung mehr. Vorzugsweise werden pro Mol des aromatischen Diazoniumsalzes 10 bis 50 g, ganz besonders bevorzugt 20 bis 40 g, Katalysator eingesetzt.

Bevorzugte Katalysatoren sind Aktivkohle, Silicagel und Aluminiumoxid. Auch Mischungen verschiedener Katalysatoren können verwendet werden. Der Katalysator wird nach Beendigung der Reaktion abgetrennt, z.B. abfiltriert, und das Reaktionsgemisch in bekannter Weise aufgearbeitet.

Dem Reaktionsgemisch kann ein an sich bekanntes Tensid, z.B. ein nichtionogenes Tensid oder Aniontensid, oder ein Entschäumer bzw. Schaumverhütungsmittel zugesetzt werden, um eine bessere Verteilung der Komponenten zu gewährleisten und Schaumbildung bei der Stickstoffenwicklung zu verhindern. Auch Gemische mehrerer dieser Stoffe können zugesetzt werden. Der Zusatz dieser Mittel erfolgt zweckmässigerweise bereits vor Beginn der eigentlichen Reaktion.

Das erfindungsgemässe Verfahren bietet gegenüber dem Stand der Technik mehrere Vorteile. Beispielsweise setzt die Zersetzung der Diaryldiazosulfid-Zwischenstufe unter Stickstoffentwicklung bereits bei tiefer Temperatur ein, wodurch die Bildung von Phenolen vermieden wird. Die Ausbeute an Diarylsulfiden der Formel IV wird gegenüber der ohne Katalysator durchgeführten Methode deutlich erhöht. Eine Belastung der Umwelt durch Schwermetalle oder Schwermetallsalze entfällt. Ferner wird eine Belastung von Fabrikationsabwässern durch toxische oder biologisch schwer abbaubare phenolische Verbindungen durch die erfindungsgemässe Arbeitsweise wesentlich herabgesetzt.

Die Erkenntnis, dass die Reaktion zwischen einem aromatischen Diazoniumsalz und einem Arenthiol im alkalischen Medium durch nichtmetallische Festsubstanzen mit einer spezifischen Oberfläche von gleich oder grösser als 0,02 $m^2/cm^3$, wie z.B. Aktivkohlen, Molekularsieben, Ionenaustauschern, Silicagel etc., katalysiert wird, muss als in hohem Masse überraschend bezeichnet werden, da bisher davon ausgegangen werden musste, dass Umsetzungen, die, wie die Sandmeyer-Reaktion, unter Ersatz der Diazoniumgruppe verlaufen, nur durch Metalle, insbesondere Kupfer, und lösliche Metallsalze, insbesondere Kupfersulfat, katalysiert werden, wobei das Metall bzw. Metallion in irgend einer Weise, z.B. durch Bildung von Komplexen, in den Ablauf der Reaktion eingreift.

Die nach dem erfindungsgemässen Verfahren herstellbaren Diarylsulfide der Formel IV

$$Ar^1\text{-}S\text{-}Ar^2 \qquad (IV),$$

worin $Ar^1$ und $Ar^2$ die bereits genannte Bedeutung besitzen, sind wertvolle Zwischenprodukte für verschiedene Anwendungsbereiche, beispielsweise zur Herstellung von Thioxanthon-Derivanten, die u.a. für die Pharmaindustrie oder als Photosensibilisatoren für UV-härtende Lacke von Bedeutung sind.

*Beispiel 1*

144 g 2-Aminobenzoesäure (Anthranilsäure) werden in einer Mischung von 1,5 l Wasser und 181 ml 32% iger Salzsäure mit einer Lösung von 74,5 g Natriumnitrit in 200 ml Wasser bei 0 bis 5°C diazotiert.

Nach Entfernen überschüssiger salpetriger Säure mit Amidosulfonsäure dosiert man die erhaltene Lösung des Diazoniumsalzes bei 3 bis 8°C zu einem gut gerührten Gemisch von 144,6 g 4-Chlorthiophenol und 35 g Aktivkohle (spezifische Oberfläche nach BET 750 $m^2/g$) in 5 l Wasser und 200 ml 10N Natronlauge. Die Stickstoffentwicklung setzt sofort ein. Man rührt 2 h bei 3 bis 8°C sowie 1 h bei 20°C nach und filtriert anschliessend vom Katalysator ab. Die alkalische Reaktionslösung wird mit 200 ml 10N Salzsäuze auf pH 1 gestellt, wobei das Reaktionsprodukt in kristalliner Form ausfällt. Nach Filtration, Neutralwaschen der Paste und Trocknung erhält man 245,5 g (2-Carboxyphenyl)-(4-chlorphenyl)-sulfid mit einem Reingehalt von 93% entsprechend 228 g (2-Carboxyphenyl)-(4-chlorphenyl)-sulfid 100% (86% der Theorie, bezogen auf 4-Chlorthiophenol).

Aus der erhaltenen Rohware kann durch bekannte Cyclisierung in guter Qualität und Ausbeute 2-Chlorthioxanthon erhalten werden.

*Vergleichsbeispiel*

Die nach den Angaben des Beispiels 1 hergestellte Lösung des Diazoniumsalzes der 2-Aminobenzoesäure wird zu einer Mischung von 144,6 g 4-Chlorthiophenol, 200 ml 10N Natronlauge und 1 l Wasser gegeben, wobei eine Reaktionstemperatur von 50°C eingehalten werden muss, um die Stickstoffentwicklung einzuleiten und eine sofortige Zersetzung der explosionsgefährlichen Diaryldiazosulfid-Zwischenstufe zu gewährleisten. Man rührt 2 h nach, filtriert von 4,4'-Dichlordiphenyldisulfid (61 g nach Trocknung) ab und isoliert das Reaktionsprodukt in Analogie zu Beispiel 1 durch Ansäuern des alkalischen Filtrats mit 10N Salzsäure bei pH 1. Ausbeute nach Trocknung: 188 g.

Gehalt an (2-Carboxyphenyl)-(4-chlorphenyl)-sulfid 72%. Ausbeute in % der Theorie: 51%.

*Beispiel 2*

Bei einer Wiederholung des Beispiels 1 wird die Aktivkohle durch 35 g eines handelsüblichen Perlite-Filterhilfsmittels mit einer spezifischen Oberfläche von ca. 0,35 $m^2/cm^3$ ersetzt. Ausserdem werden dem vorgelegten Gemisch zusätzlich 3 ml eines handelsüblichen Entschäumers («Entschäumer 7800 neu» der Fa. Bayer AG, Leverkusen) zugesetzt.

Bei einer sehr guten Raum/Zeit-Ausbeute erhält man eine sehr gute Produktausbeute von 86% (2--Carboxyphenyl)-(4-chlorphenyl)-sulfid.

Die nachfolgenden Beispiele 3 bis 19 werden analog den Beispielen 1 oder 2 durchgeführt. Dabei sind jeweils unter a) und b) die Ausgangsprodukte, unter c) der Katalysator, unter d) Lösungsmittel und unter e) die Ausbeute des Endproduktes angegeben.

*Beispiel 3*

a) 2-Aminobenzoesäure
b) 4-Chlorbenzolthiol
c) Silicagel
d) Wasser
e) 85% d.Th. (2-Carboxyphenyl)-(4-chlorphenyl)--sulfid

*Beispiel 4*

a) 2-Aminobenzoesäure
b) 4-Chlorbenzolthiol
c) Aluminiumoxid, spezifische Oberfläche nach BET 100 $m^2/g$
d) Wasser
e) 84% d.Th. (2-Carboxyphenyl)-(4-chlorphenyl)--sulfid

*Beispiel 5*

a) 2-Aminobenzoesäure
b) 4-Chlorbenzolthiol
c) Montmorillonit
d) Wasser
e) 86% d.Th. (2-Carboxyphenyl)-(4-chlorphenyl)--sulfid

*Beispiel 6*

a) 2-Aminobenzoesäure
b) 4-Chlorbenzolthiol
c) Zeolith
d) Wasser
e) 85% d.Th. (2-Carboxyphenyl)-(4-chlorphenyl)--sulfid

*Beispiel 7*

a) 2-Aminobenzoesäure
b) Benzolthiol
c) Aktivkohle, spezifische Oberfläche nach BET 750 $m^2/g$
d) Wasser
e) 94% d.Th. Phenyl-2-carboxyphenyl-sulfid

*Beispiel 8*

a) 2-Aminobenzoesäure
b) 4-Methylbenzolthiol
c) Aktivkohle, spezifische Oberfläche nach BET 750 $m^2/g$
d) Wasser
e) 86% d.Th. (2-Carboxyphenyl)-(4-methylphenyl)-sulfid

*Beispiel 9*

a) 2-Aminobenzoesäure
b) 4-Brombenzolthiol
c) Aktivkohle, spezifische Oberfläche nach BET 1400 $m^2/g$
d) Wasser
e) 87% d.Th. (2-carboxyphenyl)-(4-bromphenyl)--sulfid

*Beispiel 10*

a) 2-Aminobenzoesäure
b) 4-Fluorbenzolthiol
c) Aktivkohle, spezifische Oberfläche nach BET 750 $m^2/g$
d) Wasser
e) 90% d.Th. (2-Carboxyphenyl)-(4-fluorphenyl)--sulfid

*Beispiel 11*

a) 2-Aminobenzoesäure
b) 4-Chlor-2,5-dimethylbenzolthiol
c) Aktivkohle, spezifische Oberfläche nach BET 750 $m^2/g$
d) Wasser
e) 84% d.Th. (2-Carboxyphenyl)-(4-chlor-2,5-dimethylphenyl)-sulfid

*Beispiel 12*

a) 2-Aminobenzoesäure
b) 4-Chlorbenzolthiol
c) Stark saurer Ionenaustauscher ®Lewatit SC 104 der Fa. Bayer AG
d) Wasser
e) 77% d.Th. (2-Carboxyphenyl)-(4-chlorphenyl)--sulfid

*Beispiel 13*

a) 2-Aminobenzoesäure
b) 4-Chlorbenzolthiol
c) Glaspulver (Durchmesser 7,5 bis 33 µm)
d) Wasser
e) 80% d.Th. (2-Carboxyphenyl)-(4-chlorphenyl)--sulfid

*Beispiel 14*

a) 2-Amino-4-chlor-benzoesäure
b) Benzolthiol
c) Aktivkohle, spezifische Oberfläche nach BET 750 $m^2/g$
d) Wasser
e) 88% d.Th. Phenyl-(2-carboxy-5-chlor-phenyl)--sulfid

*Beispiel 15*

a) 2-Amino-4-fluor-benzoesäure
b) Benzolthiol
c) Aktivkohle, spezifische Oberfläche nach BET 750 $m^2/g$
d) Wasser
e) 87% d.Th. Phenyl-(2-carboxy-5-fluor-phenyl)--sulfid

*Beispiel 16*

a) 2-Amino-4-nitro-benzoesäure
b) Benzolthiol
c) Silicagel
d) Wasser
e) 80% d.Th. Phenyl-(2-carboxy-5-nitro-phenyl)--sulfid

*Beispiel 17*

a) Anilin
b) Benzolthiol
c) Aktivkohle, spezifische Oberfläche nach BET 750 $m^2/g$
d) Wasser/Toluol (Gewichtsverhältnis: 2:1)
e) 84% d.Th. Diphenylsulfid

*Beispiel 18*

a) Anilin
b) Benzolthiol
c) Aktivkohle, spezifische Oberfläche nach BET 750 $m^2/g$
d) Wasser/Chlorbenzol (Gewichtsverhältnis: 2:1)
e) 82% d.Th. Diphenylsulfid

*Beispiel 19*

a) 4-Aminobenzoesäure
b) Benzolthiol
c) Aktivkohle, spezifische Oberfläche nach BET 750 $m^2/g$
d) Wasser/Ethanol (Gewichtsverhältnis: 4:1)
e) 81% d.Th. Phenyl-(2-carboxyphenyl)-sulfid.

**Patentansprüche**

1. Verfahren zur Herstellung von Diarylsulfiden durch Umsetzung eines aromatischen Diazoniumsalzes in alkalischem Medium mit einem Arenthiol in Anwesenheit eines Katalysators, dadurch gekennzeichnet, dass als Katalysator eine nichtmetallische Festsubstanz mit einer spezifischen Oberfläche von gleich oder grösser als 0,02 $m^2/cm^3$ verwendet wird.

2. Verfahren nach Anspruch 1, dadurch gekennzeichnet, dass die Festsubstanz eine spezifische Oberfläche von gleich oder grösser als 0,06 $m^2/cm^3$ besitzt.

3. Verfahren nach Anspruch 1 und/oder 2, dadurch gekennzeichnet, dass die Festsubstanz in feinteiliger Form mit einem Teilchendurchmesser von gleich oder kleiner als 0,3 mm, vorzugsweise gleich oder kleiner als 0,1 mm, verwendet wird.

4. Verfahren nach einem oder mehreren der Ansprüche 1 bis 3, dadurch gekennzeichnet, dass die Umsetzung bei pH-Werten über 8, vorzugsweise im pH-Bereich von 9 bis 14, durchgeführt wird.

5. Verfahren nach einem oder mehreren der Ansprüche 1 bis 4, dadurch gekennzeichnet, dass die Umsetzung im Temperaturbereich von −10 bis +50°C, vorzugsweise im Temperaturbereich von 0 bis 20°C und ganz besonders bevorzugt im Temperaturbereich von 2 bis 10°C, durchgeführt wird.

6. Verfahren nach einem oder mehreren der Ansprüche 1 bis 5, dadurch gekennzeichnet, dass pro Mol aromatischem Diazoniumsalz 5 bis 60 g, vorzugsweise 10 bis 50 g, und ganz besonders bevorzugt 20 bis 40 g, nichtmetallische Festsubstanz eingesetzt werden.

7. Verfahren nach einem oder mehreren der Ansprüche 1 bis 6, dadurch gekennzeichnet, dass als nichtmetallische Festsubstanz Aktivkohle, Silicagel oder Aluminiumoxid verwendet wird.

8. Verfahren nach einem oder mehreren der Ansprüche 1 bis 7, dadurch gekennzeichnet, dass das Arenthiol und die Festsubstanz im alkalischen Medium vorgelegt und die Lösung des aromatischen Diazoniumsalzes zudosiert wird.

9. Verfahren nach einem oder mehreren der Ansprüche 1 bis 8, dadurch gekennzeichnet, dass die Umsetzung in Gegenwart eines Entschäumers oder eines Schaumverhütungsmittels durchgeführt wird.

## Claims

1. Process for the preparation of diaryl sulphides by reaction of an aromatic diazonium salt in alkaline medium with an arenethiol in the presence of a catalyst, characterized in that a non-metallic solid with a specific surface of greater than or equal to 0.02 $m^2/cm^3$ is used as catalyst.

2. Process as claimed in Claim 1, characterized in that the solid has a specific surface of greater than or equal to 0.06 $m^2/cm^3$.

3. Process as claimed in Claim 1 and/or 2, characterized in that the solid is used in finely divided form with a particle diameter of less than or equal to 0.3 mm, preferably less than or equal to 0.1 mm.

4. Process as claimed in one or more of Claims 1 to 3, characterized in that the reaction is carried out at pH values of greater than 8, preferably in the pH range from 9 to 14.

5. Process as claimed in one or more of Claims 1 to 4, characterized in that the reaction is carried out in the temperature range of −10 to +50°C, preferably in the temperature range from 0 to 20°C and very particularly preferably in the temperature range from 2 to 10°C.

6. Process as claimed in one or more of Claims 1 to 5, characterized in that 5 to 60 g, preferably 10 to 50 g, and very particularly preferably 20 to 40 g, of non-metallic solid are employed per mol of aromatic diazonium salt.

7. Process as claimed in one or more of Claims 1 to 6, characterized in that activated charcoal, silica gel or aluminium oxide is used as the non-metallic solid.

8. Process as claimed in one or more of Claims 1 to 7, characterized in that the arenethiol and the solid are initially introduced in alkaline medium and the solution of the aromatic diazonium salt is metered in.

9. Process as claimed in one or more of Claims 1 to 8, characterized in that the reaction is carried out in the presence of an anti-foam agent or a foam inhibitor.

## Revendications

1. Procédé pour préparer des sulfures de diaryles par réaction d'un sel de diazonium aromatique, en milieu alcalin, avec un arène-thiol en présence d'un catalyseur, procédé caractérisé en ce qu'on utilise, comme catalyseur, une substance solide non métallique qui a une surface spécifique au moins égale à 0,02 $m^2/cm^3$.

2. Procédé selon la revendication 1, caractérisé en ce que la substance solide a une surface spécifique au moins égale à 0,6 $m^2/cm^3$.

3. Procédé selon l'une des revendications 1 et 2, caractérisé en ce que la substance solide est utilisée sous une forme finement divisée et a un diamètre particulaire au plan égal à 0,3 mm, de préférence au plus égal à 0,1 mm.

4. Procédé selon l'une quelconque des revendications 1 à 3, caractérisé en ce qu'on effectue la réaction à un pH supérieur à 8, de préférence dans l'intervalle de pH allant de 9 à 14.

5. Procédé selon l'une quelconque des revendications 1 à 4, caractérisé en ce qu'on effectue la réaction dans l'intervalle de température allant de −10 à +50°C, de préférence dans l'intervalle de température allant de 0 à 20°C et, plus particulièrement, dans l'intervalle de température allant de 2 à 10°C.

6. Procédé selon l'une quelconque des revendications 1 à 5, caractérisé en ce qu'on met en jeu, par mole du sel de diazonium aromatique, de 5 à 60 g de la substance solide non métallique, de préférence de 10 à 50 g ou, mieux encore, de 20 à 40 g.

7. Procédé selon l'une quelconque des revendications 1 à 6, caractérisé en ce qu'on utilise, comme substance solide non métallique, du charbon actif, du gel de silice ou de l'alumine.

8. Procédé selon l'une quelconque des revendications 1 à 7, caractérisé en ce qu'on place dès le départ dans le récipient réactionnel l'arène-thiol et la substance solide dans le milieu alcalin et on fait arriver progressivement la solution du sel de diazonium aromatique.

9. Procédé selon l'une quelconque des revendications 1 à 8, caractérisé en ce qu'on effectue la réaction en présence d'un antimousse ou d'un agent empêchant la formation d'une mousse.